# EUROPEAN PATENT APPLICATION

(11) **EP 3 404 117 A1**
(43) Date of publication of application: **21.11.2018**
(21) Application number: 17001515.0
(22) Date of filing: 11.09.2017
(51) Int. Cl.: C12Q 1/6872, C12N 15/10

(54) **A METHOD USING IPLEX AND MALDI-TOF MASS SPECTOMETRY TO DETECT VARIANTS OF GENETIC BARCODES AND ITS APPLICATION**

(30) Priority: 12.09.2016 PL 41868316
(71) Applicant: Uniwersytet Gdanski, 80-309 Gdansk (PL)
(72) Inventor: Bernat - Wójtowska, Agnieszka, 80-180 Gdansk (PL); Savatier, Pierre, 69002 Lyon (FR)
(74) Representative: Matyka, Malgorzata

(57) **Abstract**

The invention concerns the method using iPLEX MALDI-TOF mass spectrometry for the detection of genetic variants of barcodes. The invention also concerns the method for detecting variants of genetic barcodes introduced into cells / tissues or organisms and/or for tracing selected cell populations at time and / or progeny of a single cell and for studying clonal evolution during the process of tumorgenesis and / or cells tracking during both *in vitro* and *in vivo* differentiation and organogenesis processes.

## Description

The subject matter of the invention is a method using MALDI-TOF and iPLEX mass spectrometry technology for the detection of genetic barcodes and its application.

Known and hitherto methods of identifying randomly generated genetic barcodes in cell populations, based on sequencing methods, are time consuming, laborious and require a large amount of IT data to be analyzed.

Unlike random libraries, thanks to the designing of barcodes and introduction of specific barcodes of known sequence, it is possible to tag and trace single cells or/and whole organisms as well as their prompt identification. This is particularly important in the field of cell banking and in the work of transferring biological material among laboratories.

The aim of the invention is to develop a research plan / algorithm leading to the detection of genetic barcodes in cells.

The usage of MALDI-TOF mass spectrometry and iPLEX technology to detect variants of genetic barcodes introduced into cells / tissues / organisms will allow for:
- genetic identification of cells / tissues / organisms and their prompt identification/ authentication
- tracking at a given time selected cell populations and / or the progeny of a single cell
- studying clonal evolution during the process of carcinogenesis or cells tracking during the differentiation and organogenesis processes both *in vitro* and *in vivo*

The advantage of this invention is prompt and simple detection of genetic barcodes, leading to the identification of cells / cells carrying a known / specified barcode variant in populations.

The subject matter of the invention is a method using MALDI-TOF and iPLEX mass spectrometry for the detection of genetic variants of barcodes, which comprises the following phases:
- molecular cloning of genetic barcodes into the expression vector pGAE-CAG-GFP,
- formation of 5 pairs of barcodes, with each pair creating a single artificial polymorphic site,
- performing first iPLEX MALDI-TOF MS reaction,
- conducting infection of mouse embryonic stem cells,
- FACS-sorting of GFP + cells and conducting mouse embryonic stem cells culture,
- performing second iPLEX MALDI-TOF MS reaction,
- implanting selected cells into early embryo environment and culturing them for 48 hours,
- performing third iPLEX MALDI-TOF MS reaction.

Method of cloning the following barcoded sequences into pGAE-CAG-GFP expression vector:
BC07_1_a 
GAGTAAGCATCTCGATGCAAATTGGTGGAACAGTGTAAAACGACGGCCAGTGAG
AT
BC07_1_b 
ATCTCACTGGCCGTCGTTTTACACTGTTCCACCAATTTGCATCGAGATGCTTACTC
TGCA
BC07_2_a 
GAGTAACCATCTCGATGCAAATTGGTGGAACAGTGTAAAACGACGGCCAGTGAG
AT
BC07_2_b 
ATCTCACTGGCCGTCGTTTTACACTGTTCCACCAATTTGCATCGAGATGGTTACTC
TGCA
BC44_1_a 
GAGTAAAAATCTCGATGCAAATTGGTGGAACAGTGTAAAACGACGGCCAGTGAG
AT
BC44_1_b 
ATCTCACTGGCCGTCGTTTTACACTGTTCCACCAATTTGCATCGAGATTTTTACTC
TGCA
BC44_2_a 
GAGTAAATATCTCGATGCAAATTGGTGGAACAGTGTAAAACGACGGCCAGTGAG
AT
BC44_2_b 
ATCTCACTGGCCGTCGTTTTACACTGTTCCACCAATTTGCATCGAGATATTTACTC
TGCA
BC45_1_a 
GAGTAAACATCGCGATGCAAATTGGTGGAACAGTGTAAAACGACGGCCAGTGAG
AT
BC45_1_b 
ATCTCACTGGCCGTCGTTTTACACTGTTCCACCAATTTGCATCGCGATGTTTACTC
TGCA
BC45_2_a 
GAGTAAACATCCCGATGCAAATTGGTGGAACAGTGTAAAACGACGGCCAGTGAG
AT
BC45_2_b 
ATCTCACTGGCCGTCGTTTTACACTGTTCCACCAATTTGCATCGGGATGTTTACTC
TGCA
BC46_1_a 
GAGTAAACATCTAGATGCAAATTGGTGGAACAGTGTAAAACGACGGCCAGTGAG
AT
BC46_1_b 
ATCTCACTGGCCGTCGTTTTACACTGTTCCACCAATTTGCATCTAGATGTTTACTC
TGCA
BC46_2_a 
GAGTAAACATCTGGATGCAAATTGGTGGAACAGTGTAAAACGACGGCCAGTGAG
AT
BC46_2_b 
ATCTCACTGGCCGTCGTTTTACACTGTTCCACCAATTTGCATCCAGATGTTTACTC
TGCA
BC47_1_a 
GAGTAAACATCTCGATGCAAATTGGTAGAACAGTGTAAAACGACGGCCAGTGAG
AT
BC47_1_b 
ATCTCACTGGCCGTCGTTTTACACTGTTCTACCAATTTGCATCGAGATGTTTACTC
TGCA
Method where artificial polymorphic sites are developed, listed in Table 1.
Method for obtaining plasmid DNA in the form of 10 plasmids:
pGAE-CAG-GFP-BC1 (abbrevation pBC1)
pGAE-CAG-GFP-BC2 (abbrevation pBC2)
pGAE-CAG-GFP-BC3 (abbrevation pBC3)
pGAE-CAG-GFP-BC4 (abbrevation pBC4)
pGAE-CAG-GFP-BC5 (abbrevation pBC5)
pGAE-CAG-GFP-BC6 (abbrevation pBC6)
pGAE-CAG-GFP-BC7 (abbrevation pBC7)
pGAE-CAG-GFP-BC8 (abbrevation pBC8)
pGAE-CAG-GFP-BC9 (abbrevation pBC9)
pGAE-CAG-GFP-BC10 (abbrevation pBC10)

Method which is used to detect variants of genetic barcodes introduced into cells / tissues or organisms.
Method which is used for tracking at a given time selected cell populations and / or the progeny of a single cell.
Method which is used for studying clonal evolution during the process of carcinogenesis or cells tracking during the differentiation and organogenesis processes both *in vitro* and *in vivo.* Usage of the method outlined above to detect variants of genetic barcodes introduced into cells / tissues or organisms.
Usage of the method outlined above to track at a given time selected cell populations and / or the progeny of a single cell.
Usage of the method described above for studying clonal evolution during the process of carcinogenesis or cells tracking during the differentiation and organogenesis processes both *in vitro* and *in vivo.*

Description of figures, diagrams and tables:
**Fig.1** - presents the map of pGAECAFGFP vector before cloning
**Fig.2** - presents the sequence of pGAECAFGFP vector before cloning
**Fig.3** - presents the photo of agarose gel: separation of PCR products
**Fig.4** - presents the map of pGAECAFGFP vector after cloning of core barcode sequence
**Fig.5** - presents the sequence of pGAECAFGFP vector after cloning of core barcode sequence
**Fig.6** - presents the sequences introduced for designing of iPLEX reaction
**Fig.7** - presents the report of AssayDesign BC1-10

**Legend:**

| SNP_ID SNP IDENTIFICATOR | UEP_MASS MASS OF UNEXTENDED PRIMER |
|---|---|
| 2nd-PCRP SECOND PRIMER FOR PCR REACTION | UEP_SEQ SEQUENCE OF UNEXTENDED PRIMER |
| 1st-PCRP FIRST PRIMER FOR PCR REACTION | EXT1_CALL 1st NUCLEOTIDE RECOGNIZED IN EXTENTION REACTION |
| AMP_LEN AMPLICON LENGHT | EXT1_MASS MASS OF 1ST PRODUCT OF EXTENTION REACTION (MASS OF EXTENDED PRIMER) |
| Tm(NN) MELTING TEMPERATURE OF PRIMER | EXT2_CALL 2nd NUCLEOTIDE RECOGNIZED IN EXTENTION REACTION |
| PcGC GC CONTENT IN PRIMER | EXT2_MASS Mass of 2nd PRODUCT OF EXTENTION REACTION (MASS OF EXTENDED PRIMER) |
| UEP_DIR DIRECTION OF ENXTENDED PRIMER | |

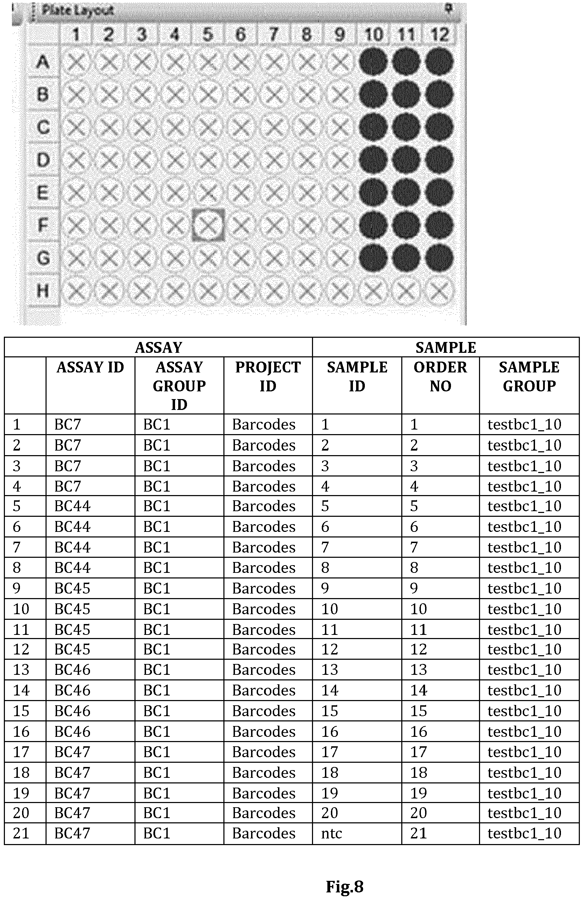

**Fig.8** - presents the experimental plan for BC1-10;

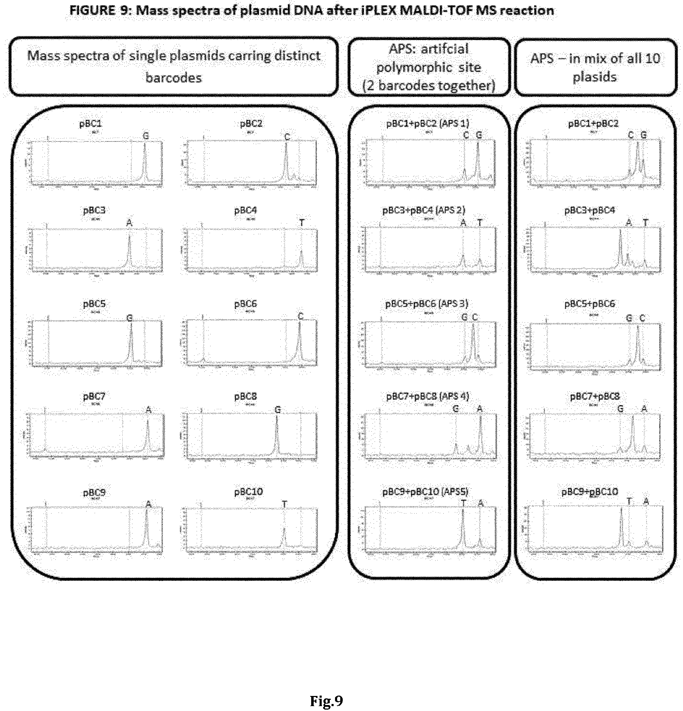

**Fig.9** - presents the mass spectra of plasmid DNA

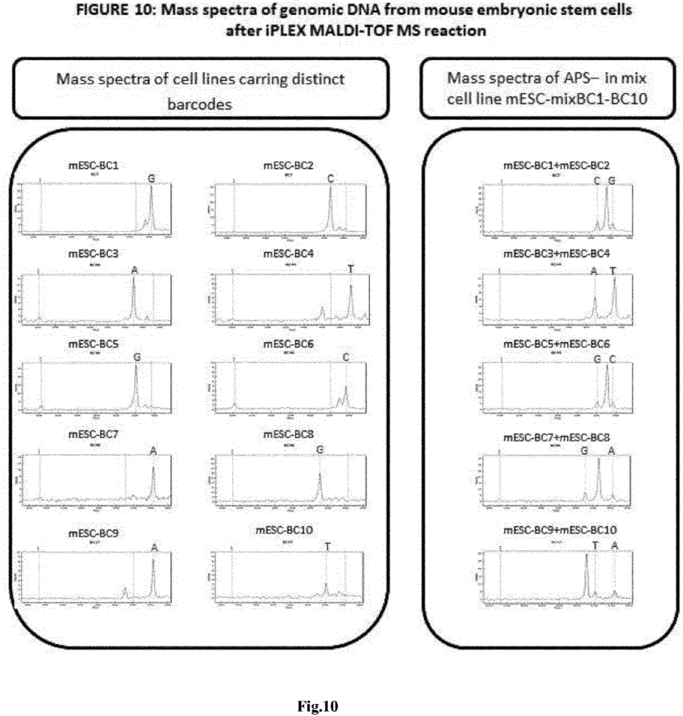

**Fig.10** - presents the mass spectra of genomic DNA isolated from mouse embryonic stem cells

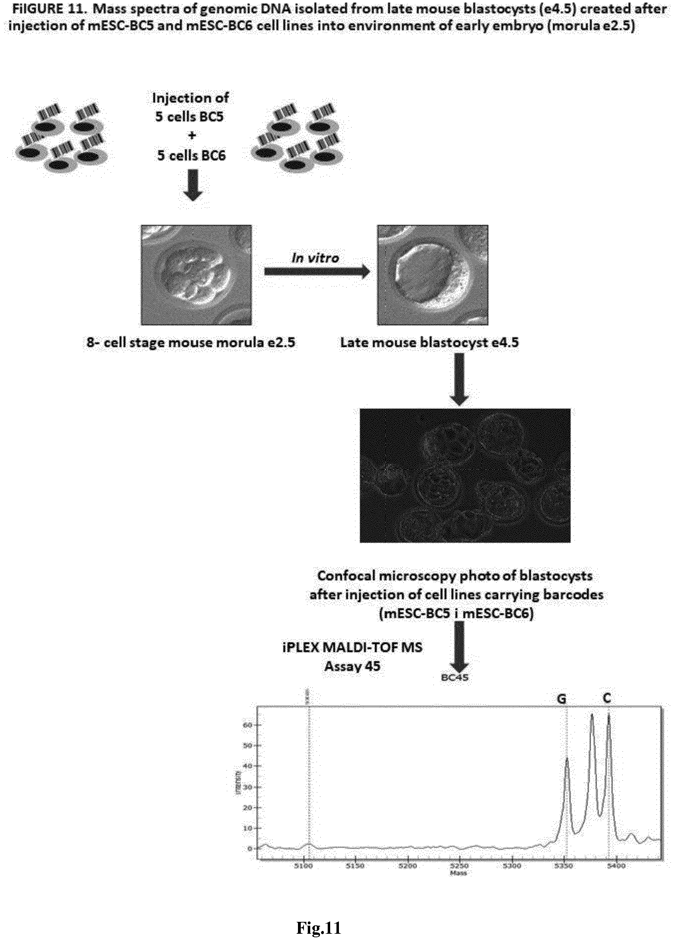

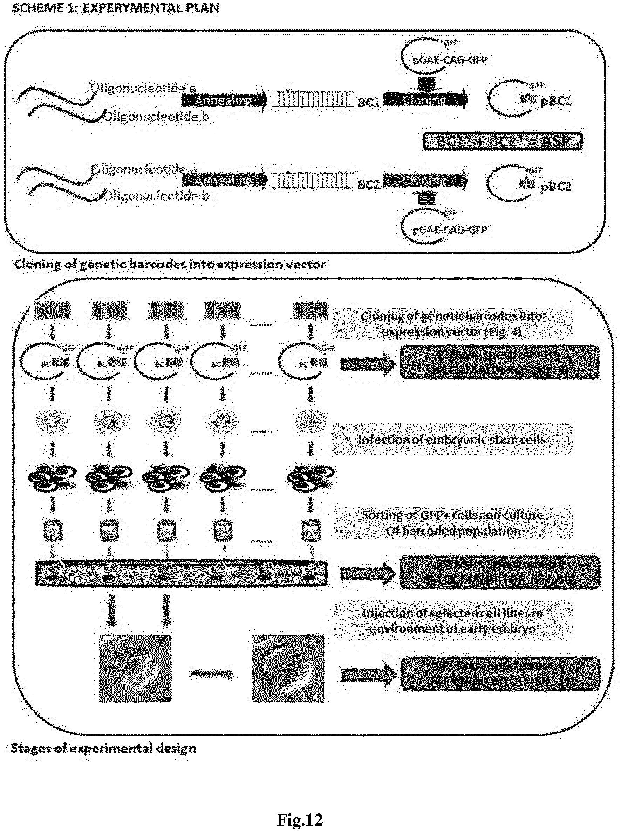

**Fig.11** - presents the barcodes BC5 and BC6 introduced into early blastocyst together with cells carrying these barcodes (mESC-BC5 and mESC-BC6)

### Diagram 1 - presents the experimental outline of the innovation

### Table 1 - presents the sequences of barcodes and artificial polimorphic site (APS - ang. Artificial Polymorphic Site)

The invention is illustrated with the following, not limiting examples:

### Example:

### Phase I:

10 single/distinct genetic labels (where "*label*" means a fragment of known DNA sequence, abbreviation BC form "*barcode*") BC1-BC10 representing variants of genetic barcodes have been designed through introduction of single-nucleotide changes into the sequence of core - barcode:
AGTAANNATCNNGATSSAAANNGGTNNAACNNTGTAAAACGACGGCCAGTGA, in N position, in a way that the library of 10 different, separated barcodes have been created. The introduced change in given position appears only once in the barcode library.

The barcodes were designed in order to create 5 pairs of barcodes, each pair having a different single-nucleotide change in the given position, thus creating a single artificial polymorphic site (APS) recognized by the iPLEX MALDI-TOF MS technology. The other barcodes in a given position carry a consensus nucleotide (diagram 1).

**Table 1:**

| | |
|---|---|
| CORE | AGTAAN1N2ATCN3N4GATS5S6AAAN7N8GGTN9N10AACN11N12TGT AAAACGACGGCCAGTGA |
| BC1 (barcode 1) | AGTAAGCATCTCGATGCAAATTGGTGGAACAGTGTAAAACGAC GGCCAGTGA |
| BC2 (barcode 2) | AGTAACCATCTCGATGCAAATTGGTGGAACAGTGTAAAACGAC GGCCAGTGA |
| APS1 | AGTAA[G/C]CATCTCGATGCAAATTGGTGGAACAGTGTAAAAC GACGGCCAGTGA |
| BC3 (barcode 3) | AGTAAAAATCTCGATGCAAATTGGTGGAACAGTGTAAAACGAC GGCCAGTGA |
| BC4 (barcode 4) | AGTAAATATCTCGATGCAAATTGGTGGAACAGTGTAAAACGAC GGCCAGTGA |
| APS2 | AGTAAA[A/T]ATCTCGATGCAAATTGGTGGAACAGTGTAAAAC GACGGCCAGTGA |
| BC5 (barcode 5) | AGTAAACATCGCGATGCAAATTGGTGGAACAGTGTAAAACGA CGGCCAGTGA |
| BC6 (barcode 6) | AGTAAACATCCCGATGCAAATTGGTGGAACAGTGTAAAACGAC GGCCAGTGA |
| APS3 | AGTAAACATC[G/C]CGATGCAAATTGGTGGAACAGTGTAAAAC GACGGCCAGTGA |
| BC7 (barcode 7) | AGTAAACATCTAGATGCAAATTGGTGGAACAGTGTAAAACGAC GGCCAGTGA |
| BC8 (barcode 8) | AGTAAACATCTGGATGCAAATTGGTGGAACAGTGTAAAACGAC GGCCAGTGA |
| APS4 | AGTAAACATCT[A/G]GATGCAAATTGGTGGAACAGTGTAAAAC GACGGCCAGTGA |
| BC9 (barcode 9) | AGTAAACATCTCGATGCAAATTGGTAGAACAGTGTAAAACGAC GGCCAGTGA |
| BC10 (barcode 10) | AGTAAACATCTCGATGCAAATTGGTTGAACAGTGTAAAACGAC GGCCAGTGA |
| | |
| APS5 | AGTAAACATCTCGATGCAAATTGGT[A/T]GAACAGTGTAAAAC |
| | GACGGCCAGTGA |

### 1. Molecular cloning of genetic barcodes into the expression vector pGAE-CAG-GFP

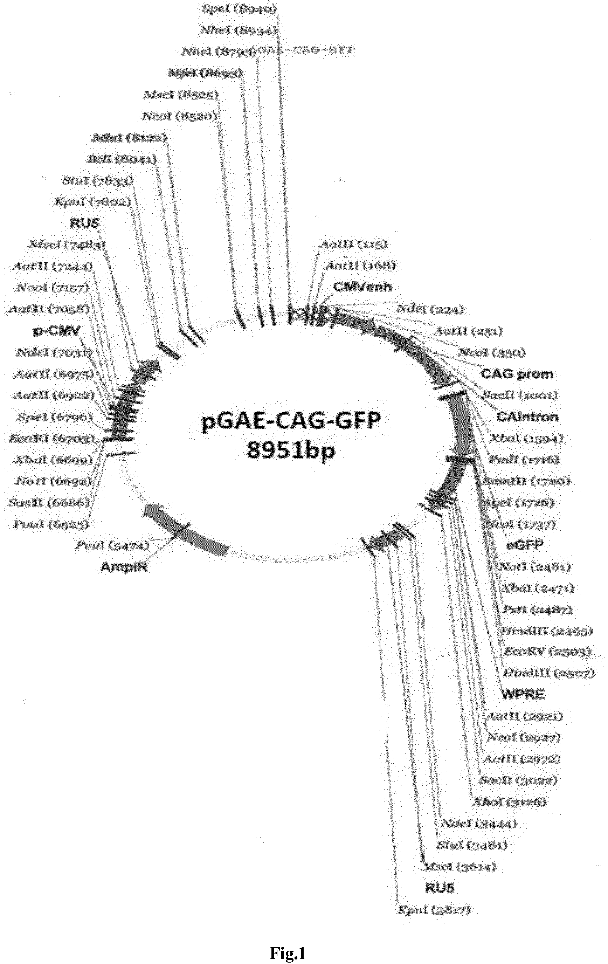

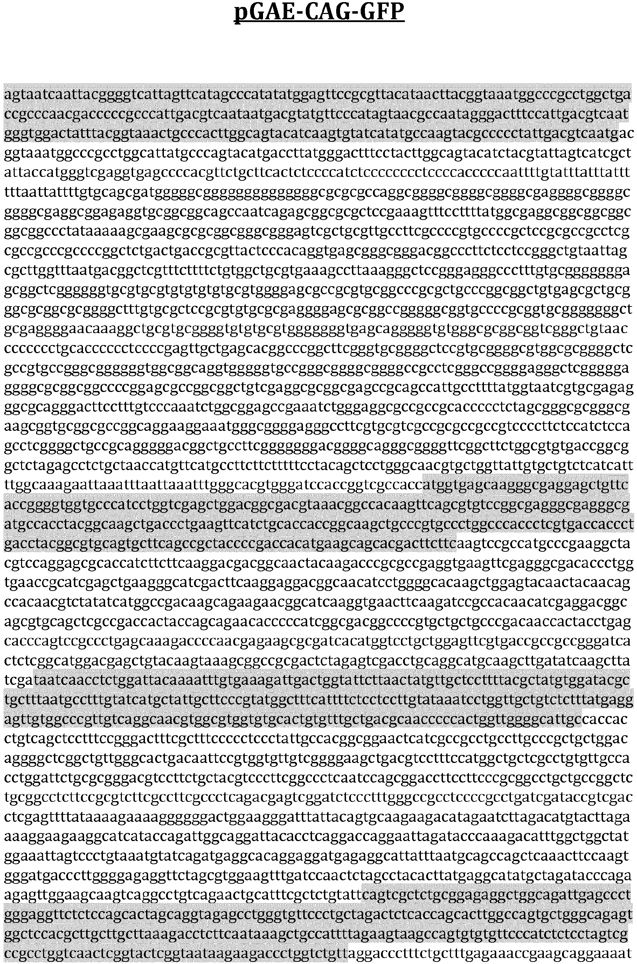

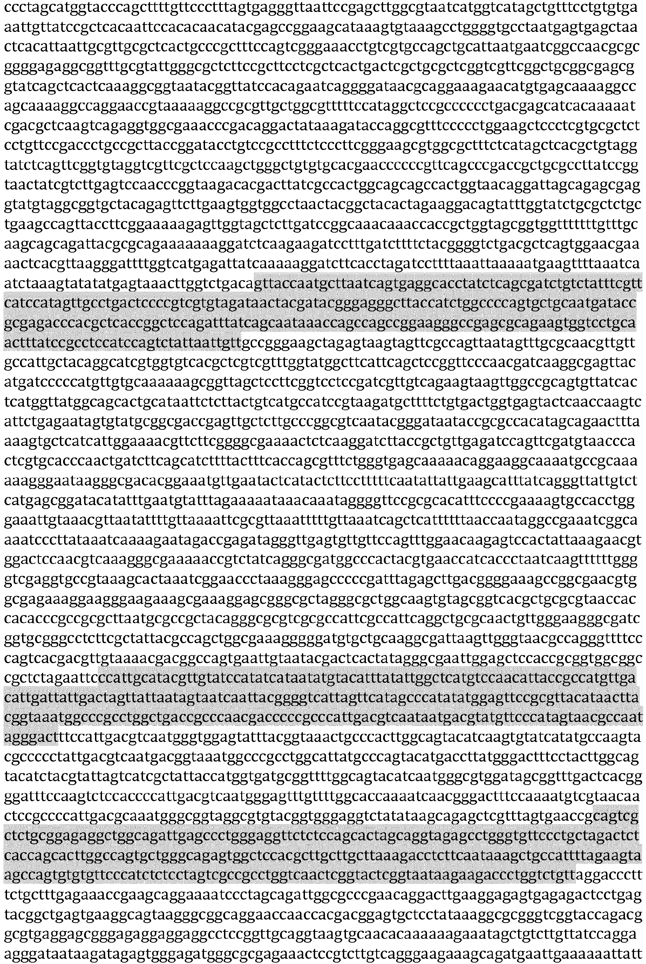

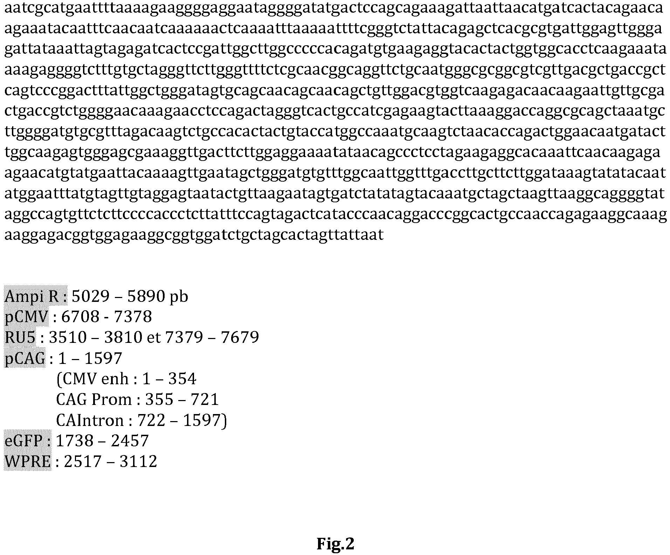

10 individual expression vectors carrying a single genetic barcode were created by molecular cloning (Fig.1, Fig. 2).

### 1a. Preparation of the expression vector to accept / clone the barcode

**i) Digestion of the vector with restriction enzymes** *PstI*/*EcoRV and dephosphorylation of 5'ends after digestion*

**Reaction mixture:**

| | Digestion of vector pGAE-CAG-GFP (stock at 1.25 ug/ul) |
|---|---|
| Vector | 2.µl (2.5 µg) |
| Buffer R* | 2µl |
| *enzyme PstI** | 1µl |
| *enzyme EcoRV** | 1µl |
| H₂O | 14µl |
| | 20µl: final volume of the reaction |
| Time/Temperature | 2h30' 37°C |
| | Defosphorylation (added to digestion reaction) |
| *Enzyme Fast AP** | 1 µl |
| Buffer AP* | 2.5 µl |
| H₂0 | 1.5 µl |
| | 25 µl: final volume of the reaction |
| Time/Temperature | 30' 37+ 5'75°C |
| * commercially available kit | |

**ii)** Purification of the reaction product: the vector (the whole reaction mixture) was purified by a commercially available column system, next the product was eluted in 120 µl ddH20 and its concentration was measured (Nanodrop). Such prepared vector was ready to accept the insert - the barcode.

### 1b. Preparation of barcodes

Synthesis of barcodes (DNA fragments) was ordered. The barcodes were synthesized as 20 single-stranded DNA fragments, each one additionally having a *PstI* restriction site at 5' end (marked red) and *EcoRV* at the 3' end (marked blue) and additionally having 5' end phosphorylated

Sequence of single-stranded fragments (oligonucleotides):
BC07_1_a 
GAGTAAGCATCTCGATGCAAATTGGTGGAACAGTGTAAAACGACGGCCAGTGAG
AT
BC07_1_b 
ATCTCACTGGCCGTCGTTTTACACTGTTCCACCAATTTGCATCGAGATGCTTACTC
TGCA
BC07_2_a 
GAGTAACCATCTCGATGCAAATTGGTGGAACAGTGTAAAACGACGGCCAGTGAG
AT
BC07_2_b 
ATCTCACTGGCCGTCGTTTTACACTGTTCCACCAATTTGCATCGAGATGGTTACTC
TGCA
BC44_1_a 
GAGTAAAAATCTCGATGCAAATTGGTGGAACAGTGTAAAACGACGGCCAGTGAG
AT
BC44_1_b 
ATCTCACTGGCCGTCGTTTTACACTGTTCCACCAATTTGCATCGAGATTTTTACTC
TGCA
BC44_2_a 
GAGTAAATATCTCGATGCAAATTGGTGGAACAGTGTAAAACGACGGCCAGTGAG
AT
BC44_2_b 
ATCTCACTGGCCGTCGTTTTACACTGTTCCACCAATTTGCATCGAGATATTTACTC
TGCA
BC45_1_a 
GAGTAAACATCGCGATGCAAATTGGTGGAACAGTGTAAAACGACGGCCAGTGAG
AT
BC45_1_b 
ATCTCACTGGCCGTCGTTTTACACTGTTCCACCAATTTGCATCGCGATGTTTACTC
TGCA
BC45_2_a 
GAGTAAACATCCCGATGCAAATTGGTGGAACAGTGTAAAACGACGGCCAGTGAG
AT
BC45_2_b 
ATCTCACTGGCCGTCGTTTTACACTGTTCCACCAATTTGCATCGGGATGTTTACTC
TGCA
BC46_1_a 
GAGTAAACATCTAGATGCAAATTGGTGGAACAGTGTAAAACGACGGCCAGTGAG
AT
BC46_1_b 
ATCTCACTGGCCGTCGTTTTACACTGTTCCACCAATTTGCATCTAGATGTTTACTC
TGCA
BC46_2_a 
GAGTAAACATCTGGATGCAAATTGGTGGAACAGTGTAAAACGACGGCCAGTGAG
AT
BC46_2_b 
ATCTCACTGGCCGTCGTTTTACACTGTTCCACCAATTTGCATCCAGATGTTTACTC
TGCA
BC47_1_a 
GAGTAAACATCTCGATGCAAATTGGTAGAACAGTGTAAAACGACGGCCAGTGAG
AT
BC47_1_b

ATCTCACTGGCCGTCGTTTTACACTGTTCTACCAATTTGCATCGAGATGTTTACTC
TGCA

In order to create double stranded fragments as inserts for cloning into the expression vector, synthesized single-stranded barcodes were subjected to annealing process (each barcode _a + each barcode_b):
Composition of the reaction mixture: 20 µl oligonucleotide_a + 20 µl oligonucleotide_b + 5 µl 10x Ligation Buffer + 5 µl H₂0
(incubation for 3 minutes at 95°C, followed by slow cooling at room temperature for 45 minutes). When the reaction was finished, the barcodes were diluted 1:20 before further use.

### |Oligonucleotide mix:

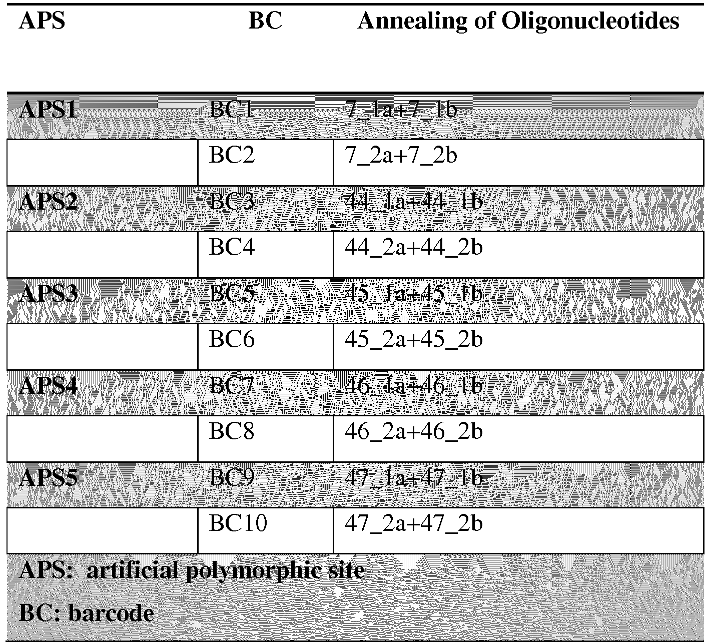

### 1c. Ligation of the prepared vector and barcodes

10 separate ligation mixtures were prepared, each containing a different barcode (BC): Composition of the ligation mixture:

| | **Ligation** |
|---|---|
| Vector Digestedwith *Psti*/*EcoRV* | 12µl |
| Ligase buffer 10x* | 2 µl |
| *Ligase* | 1.5 µl |
| PEG4000* | 1 µl |
| Barcode (BC) | 1 µl |
| H₂0 | 1.5 µl |
| | 20 µl: final volume of the reaction |
| Time/Temperature | 16h, 22°C, |
| * commercially available kit | |

### 1d. Transformation of competent E. coli cells

25 µl of commercially available competent bacteria (cfu ≥ 10⁹) were transformed with 3 µl of the ligation mixture according to the protocol provided by the manufacturer. 10 separate transformation reactions were performed for each ligation mixture separately. After transformation, bacterial cells were amplified in 1 mL of LB liquid medium and 200 µl of each mixture was plated on a solid substrate / selective antibiotic plate (ampicillin 100 µg / ml). Plates were incubated for 20 hours at 37°C.

Bacterial cells that have accepted the plasmid with a properly cloned barcode, gain ampicillin resistance.

On the next day, 3 colonies were selected from each plate in which the presence of the cloned marker was confirmed by PCR.

### 1e. PCR to confirm the correctly performed cloning of barcodes into pGAECAGGFP

Amplification of the plasmid region where cloning was aimed, confirmed the presence of a barcode and confirmed correctly performed cloning.

The expected PCR product size after cloning was 214 bp, while the size of the empty plasmid PCR product was 174 bp.

The primer sequences used for the amplification reaction were as follows:
Primer Forward: GGACGAGCTGTACAAGTAAAGC
Primer Reverse: GGCATTAAAGCAGCGTATCC

Three colonies from each plate (phase 1d) were suspended in 20 µl H2O, of which 2 µl was used for PCR. Positive control in PCR was the plasmid DNA isolated from 100 randomly selected colonies. Negative control (of the purity) - water, the empty expression plasmid pGAE-GAG-GFP was additionally amplified.

The remaining part of the bacterial suspension was cultured overnight in 1 ml of LB antibiotic liquid medium, at 37C with shaking to amplify the culture.

**Composition of the PCR mixture:**

| | For 1 reaction/matrix |
|---|---|
| Buffer 10x* | 2.5 µl |
| MgCl₂ 25mM* | 2 µl |
| dNTPs 10µM* | 0.5 µl |
| Primer F 1seq 10µM** | 1 µl |
| Primer R 1seq 10µM** | 1 µl |
| *Taq* polimerase* | 0.2 µl |
| H₂0 | 15.8 µl |
| Matrix (bacterial colony suspended in | 2 µl |
| or DNA (positive and negative control) | 100ng/2 µl |
| Volume: | 25 µl |
| * commercially available kit | |
| ** synthesis of specific primers was ordered | |

Such prepared samples (total of 33 samples) were placed in a thermocycler and the PCR reaction was carried out with the following thermal profile:
95°C 3min 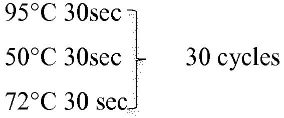
72°C 5min
10°C cooling down

### If. Electrophoresis of PCR products in agarose gel.

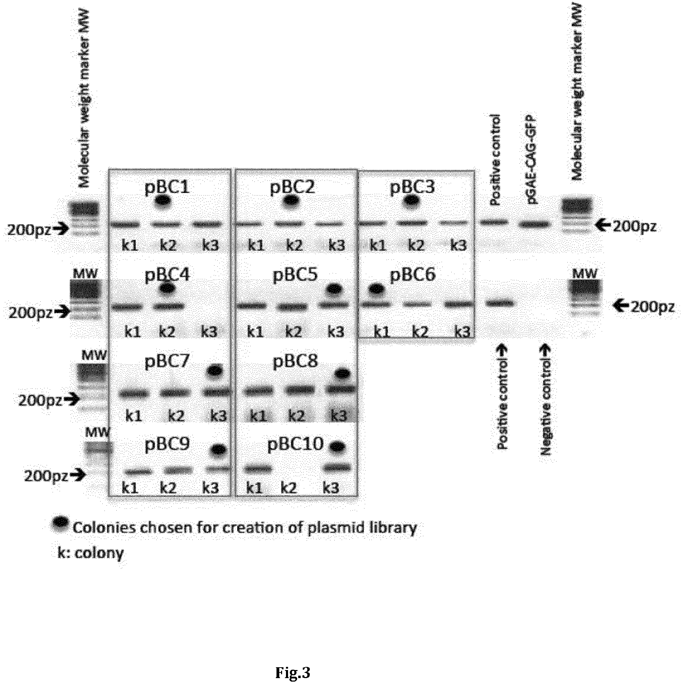

For the visualization of PCR products, 10 µl of PCR products were separated in 2.2% agarose gel with ethidium bromide (5µg / ml), after loading with 6x loading buffer (commercially available). Electrophoresis was performed for 20 minutes at 50 V, in the presence of 100bp molecular size marker (commercially available) - Fig.3.

PCR confirmed the correctness of cloning in most of colonies.

### 1g. Isolation of plasmid DNA.

From bacterial cultures started from single colonies (see 1e), single culture with correctly cloned barcode was selected for further analysis. Each culture was diluted 1:10 with fresh LB+ antibiotic medium and cultured overnight at 37°C with shaking. The plasmid DNA was isolated from each culture using a commercially available plasmid DNA isolation kit which led to obtaining 10 plasmids (expression vectors):
1.pGAE-CAG-GFP-BC1 (abbrevation pBC1)
2. pGAE-CAG-GFP-BC2 (abbrevation pBC2)
3. pGAE-CAG-GFP-BC3 (abbrevation pBC3)
4. pGAE-CAG-GFP-BC4 (abbrevation pBC4)
5. pGAE-CAG-GFP-BC5 (abbrevation pBC5)
6. pGAE-CAG-GFP-BC6 (abbrevation pBC6)
7. pGAE-CAG-GFP-BC7 (abbrevation pBC7)
8. pGAE-CAG-GFP-BC8 (abbrevation pBC8)
9. pGAE-CAG-GFP-BC9 (abbrevation pBC9)
10. pGAE-CAG-GFP-BC10 (abbrevation pBC10)

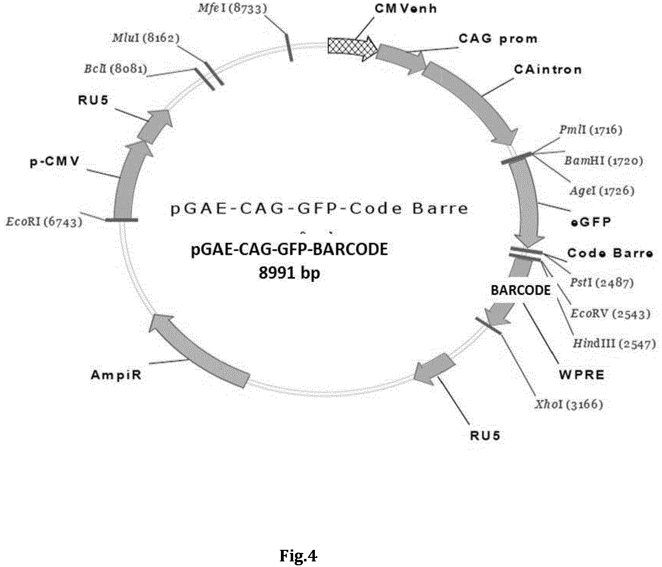

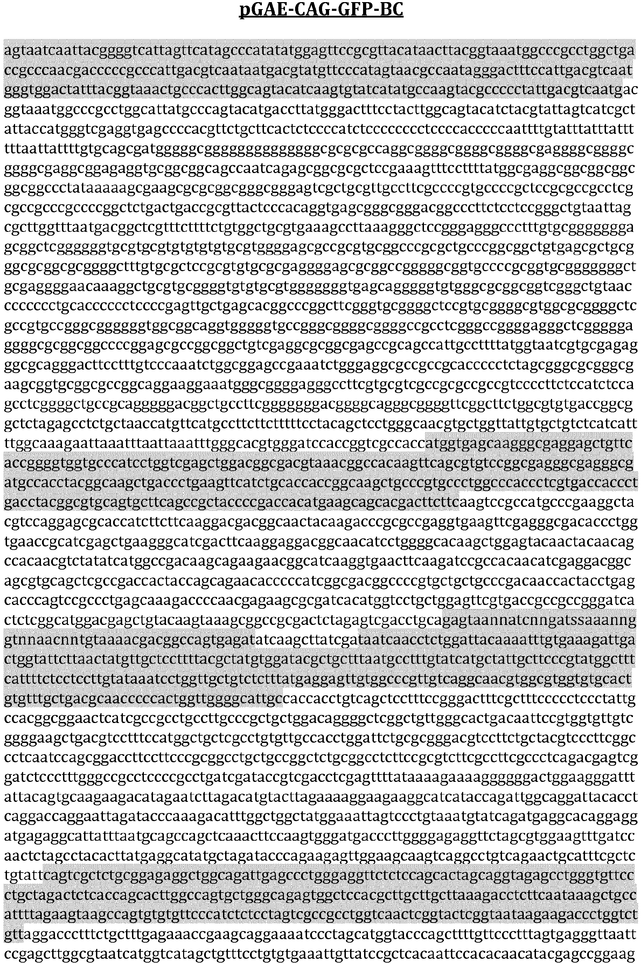

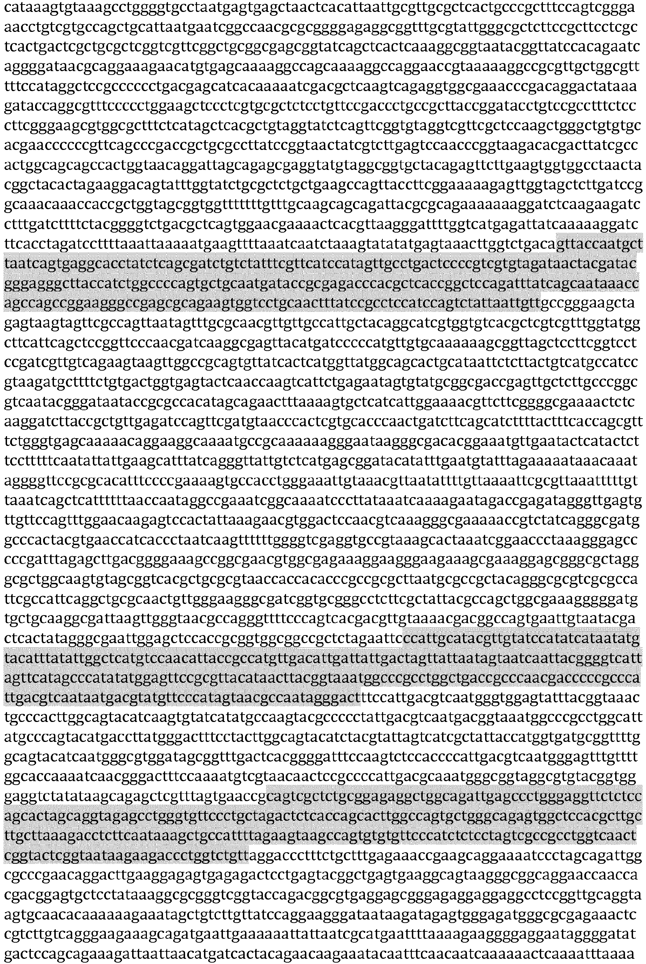

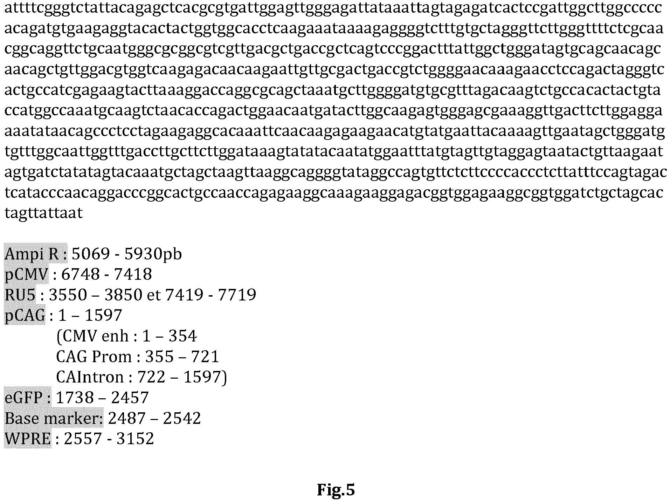

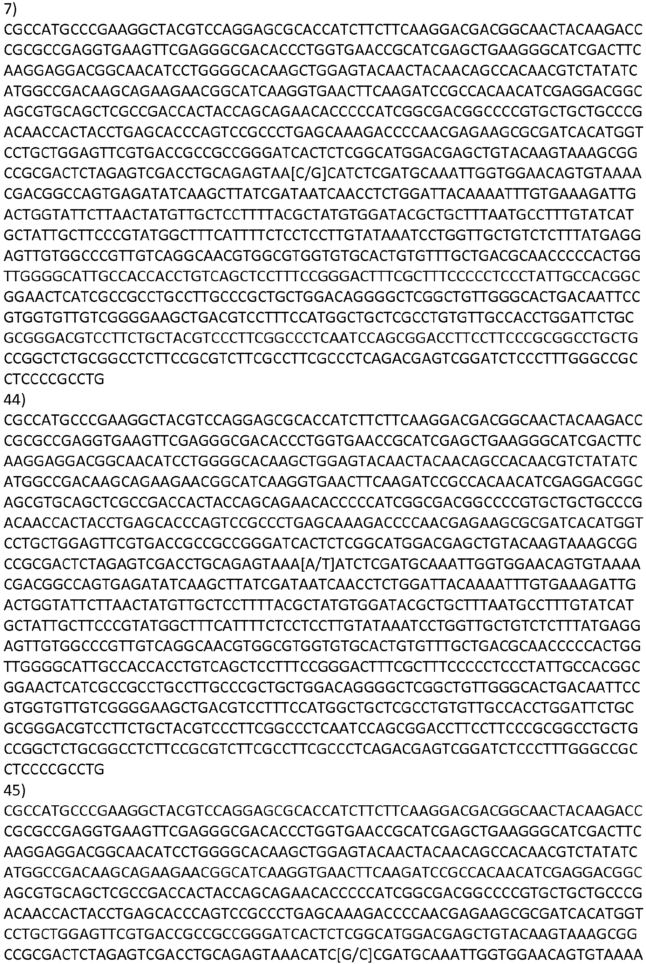

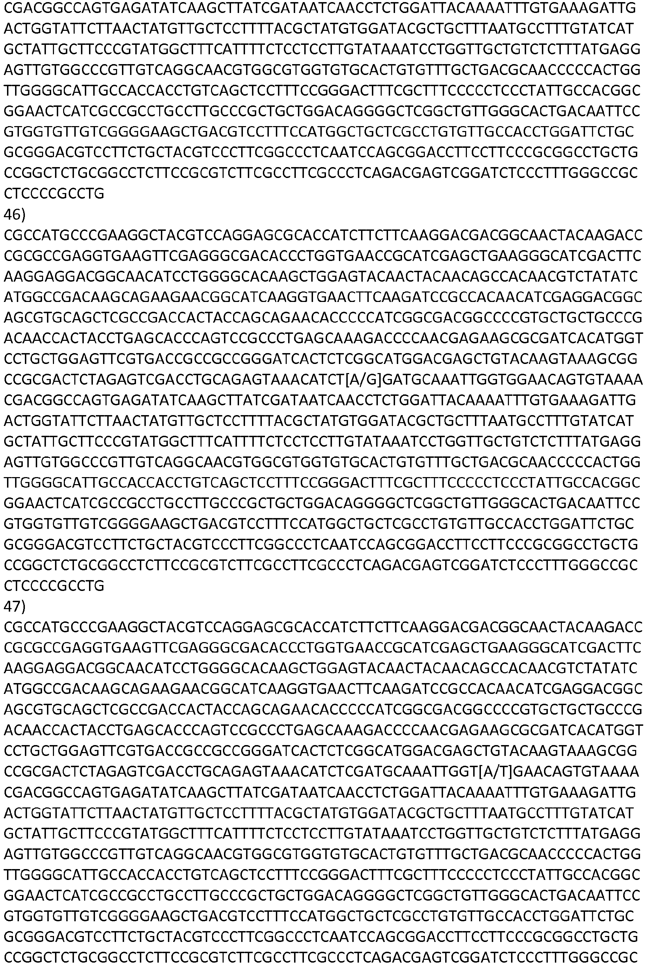

Each vector carries a defined and known genetic barcode.
Diagram of the resulting plasmids / vectors as well as the plasmid sequence after barcode cloning (on the example of core barcode) - (Fig. 4, Fig. 5).

Phase II. Project validation: Identification of Barcodes with the iPLEX MALDI-TOF MS reaction.

iPLEX technology is based on amplification of a region containing barcode sequence in PCR reaction and subsequent iPLEX reaction with specific primers (designed thanks to the Assay Design software available on https://www.agenacx.com/Tools) hybridizing next to the introduced single nucleotide change site in core barcode sequence and next to the elongated primers ("extend" reaction) with polimerse (all iPLEX GOLD reagents supplied by Agena). Elongation is terminated after single nucleotide is incorporated, exactly in place of single nucleotide change leading to the creation of products of specific, known and distinct masses. Resulting mass spectra are recorded and analyzed by Mass Array 4 (Agena) and Mass Array Analyzer (Agena).

### 2a. Designing conditions for the iPLEX MALDI-TOF MS reaction

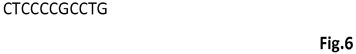

MALDI-TOF reaction data as well as primer sequences (for PCR and "extend" reactions) were generated by Assay Design Suite v.2.0 programme. In contrast to the typical genotyping reaction, partial sequences of plasmid DNA with marked APS polymorphic sites were added to the design of Assay Design programme (bookmark: "current input: file upload") and the following parameters were applied - bookmark: "organism: other, iPLEX chemistry, multiplex level: 5". The remaining parameters were unchanged (optimum settings designed by Agena) - Fig.6.

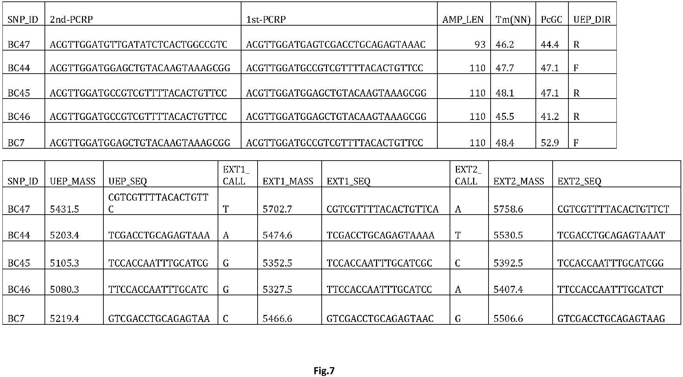

The report generated by the Assay Design programme (Excel file, Fig. 7) was used to design an experiment in the Mass Array Typer 4.0 programme, to which the information about the samples ("samples") was loaded in addition to the Assay Design data. That way a plan for the P1 experiment ("plate") was created - Fig. 7, Fig. 8.

By the generated assay design file (Figure 7): Assay 7: Detects BC1 and BC2, assay 44 detects BC3 and BC4, Assay 45 BC5 and BC6, Assay 46 BC 7 and BC8, Assay 47 BC9 and BC10.

### 2b. iPLEX MALDI_TOF MS

Molecular/analytical steps in iPLEX MALDI_TOF MS reaction are as follows: i) PCR reaction ii) SAP reaction iii) Extend reaction iv) sample conditioning v) dispensing samples on SpecrtaChip with usage of nanodispeser and vi) reading and automatic analysis in mass spectrometer (MS4)
i) Amplification - PCR reaction
The samples were 10 ng of plasmid DNA from 10single plasmids (pBC1-pBC10) and mix of pairs of plasmids constituting APS sites: pBC1+pBC2 (APS1), pBC3+pBC4 (APS2), pBC5+ pBC6 (APS3), pBC7+pBC8, pBC9+ pBC10 (APS5).
H20 was the "negative control sample'.

**PCR reaction composition:**

| | For 1 reaction/DNA sample |
|---|---|
| Bufeer 10x* | 0.5 µl |
| MgCl₂ 25mM* | 0.4 µl |
| dNTPs 10µM* | 0.1 µl |
| Primer mix F+R** | 1 µl |
| *Taq* polimerase* | 0.2 µl |
| H₂0 | 0.8 ul |
| DNA (samples) | 2 µl |
| Final volume: | 5 µl |
| * reagents set by Agena for PCR reaction is commercially available, | |
| ** synthesis of specific primers (sequences designed by Assay Design Suite 2.0) | |

Such prepared samples were placed in a thermocycler and PCR reaction proceeded with the following thermal profile:
95°C 2 minutes 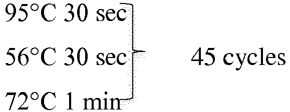
72°C 5 minutes
10°C cooling down

ii) SAP reaction
The SAP reaction is carried out to remove dNTPs which are non-incorporated during PCR. 2 µl of the SAP reaction mixture was added to the reaction mixture after PCR (5 µl).

**SAP reaction mix:**

| | for 1 reaction |
|---|---|
| SAP* Buffer | 0.17 µl |
| SAP* Enzyme | 0.3 µl |
| H₂0 | 1.53 µl |
| Sample: | 5 µl |
| : | 7 µl |
| * reagents set by Agena for SAP reaction is comercially | |

The reaction was carried out in a thermocycler under the following thermal conditions:
37° C 40 minutes
85° C 5 minutes
10° C cooling down

**iii) "Extend" reaction**
2 µl of the iPLEX reaction mixture was added to the reaction mixture after the SAP reaction.

**iPLEX ("Extend") reaction mix:**

| | For 1 reaction |
|---|---|
| iPLEX* Buffer | 0.2 µl |
| Termination Mix * | 0.2 µl |
| iPLEX Gold*Enzyme | 0.041µl |
| Extend *Primer | 0.94 µl |
| H₂0 | 0.619 µl |
| Sample: | 7 µl |
| Final Volume: | 9 µl |

Such prepared samples were placed in a thermocycler and the extend reaction proceeded with the following thermal profile:
95°C 30 sec 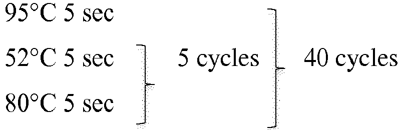
72°C 3 minutes
10°C cooling down

iv) Conditioning reaction (desalting): 41ul H₂0 was added to each sample after the extend reaction as well as dried for 10 minutes resin (0.15mg / sample) that was prepared according to recommendations of the company which was also added. The samples were shaken in a rotator for 15 minutes at room temperature. Next such prepared samples were centrifuged (4000rpm) for 15 minutes at room temperature.
v) The samples were then put on the SpectroChip by Nanodispenser RS1000 in an amount of 8-12ng +/- 25% of each sample. In addition, a 3-point calibrant, supplied with other reagents, was also added.
vi) MALDI-TOF MS Reaction: SpectroChip with the put samples was placed in a Mass Array MS4 machine. Assay Design file generated by Mass Array Typer 4.0 programme was loaded into ChipLinke programme together with SpectroChip serial number. MassARRAY Typer Workstation with iPLEX settings (iPEX.par) was launched, MALDI-TOF reaction was performed automatically while generated and analyzed spectra were recorded automatically. The resulting mass spectra are automatically transported to the Mass Array Typer 4.0 Analyzer, the experiment is being saved, and the mass spectra are described.

As a result of the iPLEX MALDi-TOF MS reaction, on the basis of the resulting mass spectra (Fig. 9), each genetic barcode was identified both in single plasmids pBC1, pBC2, pBC3,pBC4, pBC5, pBC6, pBC7, pBC8, pBC9, pBC10 as well as in the mixture of plasmid pairs constituting individual APS: pBC1+pBC2 (APS1), pBC3+pBC4 (APS2), pBC5+ pBC6 (APS3), pBC7+pBC8, pBC9+ pBC10 (APS5).

### Phase III: Establishment of genetically engineered cell culture.

### 3a. Production of viral particles

Production of concentrated virus particles is based on their production by 293T cells transfected beforehand by the "packaging" plasmid psPax2, the pMD.G plasmid encoding the viral envelope glycoprotein and the pGAE-CAG-GFP-BC transfer plasmid bearing the genetic barcode. The virus particles secreted into the supernatant were then concentrated using ultracentrifugation in the presence of sucrose and the virus titre determined by titration and percentage determination of GFP + cells using a flow cytometer. 10 separate viral stocks were produced for each vector carrying the barcode. This procedure is the standard procedure described in numerous publications (ref 1, 2).

### 3b. Infection of mouse embryonic stem cells and establishment of genetically engineered cells.

Stable expression of genetic barcodes in mouse embryonic stem cells (mESC) was obtained by infection of cell line E14Tg2a (ATCC CRL-1821) with virus particles, in 10 separate infection reactions. Cells were infected at a low moi-5 (moi - multiplicity of infection) which guaranteed a single site of integration into the cell genome. As a result of infections, 10 heterogeneous cell lines were generated, each expressing one variant of the genetic barcode under the control of the CAG promoter and conjugated to GFP. At the time of integration, each vector introduced a unique, recognizable hereditary genetic barcode into the host cell genome. GFP + cells (expressing the barcode) were separated from non-infected cells (GFP-) by sorting in a flow cytometer (FACS-sorting). In this way, 10 homogenous cell lines were created: mESC-BC1, mESC-BC2, mESC-BC3, mESC-BC4, mESC-BC5, mESC-BC6, mESC-BC7, mESC-BC8, mESC-BC9, mESC-BC10.

In addition to 10 separate cell lines, a mixed culture mESCmixBC1-BC10 was started by mixing equal amounts (1000 cells) of cells carrying each single marker.

After 3 passages, genomic DNA was isolated (commercially available kit) from mESC-BC1, mESC-BC2, mESC-BC3, mESC-BC4, mESC-BC5, mESC-BC6, mESC-BC7, mESC-BC8, , mESC-BC10 and mESCmixBC1-BC10. DNA concentration was measured (Nanodrop).

Phase IV: Detection of barcodes in genetically engineered cell culture using iPLEX MALDI-TOF MS

The detection of genetic barcodes in cell culture is analogous to the detection of genetic barcodes described phase II. The only distinction was to use genomic DNA in place of plasmid DNA. The isolated genomic DNA (point 3b) was used in the amount of 10ng / reaction. The molecular / analytical steps were the same as in Phase II, the only distinction in the experiment plan was to generate a P2 experiment plan which was different from P1 as far as the information on the analyzed samples is concerned.

As a result of the iPLEX MALDi-TOF MS reaction, on the basis of the resulting mass spectra (Fig. 9), each genetic barcode introduced to the cells was identified in cells lines: mESC-BC1, mESC-BC2, mESC-BC3, mESC-BC4, mESC-BC5, mESC-BC6, mESC-BC7, mESC-BC8, mESC-BC9, mESC-BC10 and in mixed cell culture mESCmixBC1-BC10 (Fig.10).

### Phase V: Detection of barcodes in environment of late blastocyst, after injection of two barcoded cells populations (mESC-BC5 and mESC-BC6) into early morula (e2.5) using iPLEX MALDI-TOF MS reaction.

Injection of 5 single mESC-BC5 and 5 single mESC-BC6 cells into murine morula (embryonic e2.5) was carried out. Embryos were grown for 48h to reach the stage of the late blastocyst (embryonic stage e4.5), then genomic DNA was extracted from the blastocyst. The detection of genetic barcodes in early blastocysts environment is analogous to the detection of genetic barcodes described in phase 2. The only difference is to use genomic DNA isolated from the blastocyst. 5 µl genomic DNA was used for the experiment. As a result of the iPLEX MALDI-TOF MS reaction, both BC5 and BC6 markers that were introduced into the blastocyst together with the cells bearing these markers (mESC-BC5 and mESC BC6) were detected. Fig. 11.
All reagents, samples, mixtures were vortexed and shortly spined down (for 30 seconds, 130000rpm) before usage and after mixing.

The description of mass spectra:

### Description of mass spectra:

- By assay design file (Fig. 7 Assay Design Report): Assay 7: Detects BC1 and BC2, Assay 44 Detects BC3 and BC4, Assay 45 BC5 and BC6, assays 46 BC 7 and BC8, assays 47 BC9 and BC10.
- UEP (unextended primer): none spectrum (absence of spectrum) indicates complete consumption of the primer during the elongation reaction
- The exact masses of primers and specific products of their extending together with their sequences as well as the primers for PCR and size of amplification products are included in the Assay Design Report BC1-10 (Fig. 7)
- Peaks with an intensity greater than 2 (y axis) and a blue dashed line parallel to the y axis passing through the peak are considered to be specific.
- The difference in peak intensity is due to unequal amount of used DNA (with small size of plasmid DNA, a marginal pipetting error leads to an increase in plasmid copy number and for genomic DNA it may suggest that more copies of an integrated barcode-carrying virus). For this reason sometimes peaks are marked with a red line, although its intensity is much greater than 2. iPLEX is an application created and used for genotyping 2 allels (ideal ratio is 1:1 DNA for 2 alleles) and the deviation from this ratio is recognized automatically by the programme algorithm as non-validated peaks and marked with red dashed line.
- In the barcodes mix, non-specific products appearing between or before specific peaks are signals from the remaining 8 barcodes which are elongated: instead of the APS-forming nucleotide, they are extended to the consensus nucleotide

### Bibliography:

1. Wianny, F., Bernat, A., Marcy, G., et al. (2008). Derivation and cloning of a novel Rhesus ES cell line stably expressing tau-GFP. Stem Cells 26, 1444-1453
2. Suter DM, Cartier L, Bettiol E, Tirefort D, Jaconi ME, Dubois-Dauphin M, Krause KH. Rapid generation of stable transgenic embryonic stem cell lines using modular lentivectors. (2006). Stem Cells, 24:615-23.

## Claims

1. The method using iPLEX MALDI-TOF mass spectrometry for the detection of genetic variants of barcodes which is **characterized by** the following phases:
- molecular cloning of genetic barcodes into the expression vector pGAE-CAG-GFP,
- formation of 5 pairs of barcodes, with each pair creating a single artificial polymorphic site,
- performing first iPLEX MALDI-TOF MS reaction,
- conducting infection of mouse embryonic stem cells,
- FACS-sorting of GFP + and culturing barcoded cells,
- performing second iPLEX MALDI-TOF MS reaction,
- implanting selected cells into early embryo environment and culturing them for 48 hours,
- performing third iPLEX MALDI-TOF MS reaction.

2. The Method according to claim 1 **characterized by** the following genetic barcodes being cloned into the pGAE-CAG-GFP expression vector:
BC07_1_a
**G**AGTAAGCATCTCGATGCAAATTGGTGGAACAGTGTAAAACGACGGCCAGTGAGAT
BC07_1_b 
ATCTCACTGGCCGTCGTTTTACACTGTTCCACCAATTTGCATCGAGATGCTTACTCTG
CA
BC07_2_a
**G**AGTAACCATCTCGATGCAAATTGGTGGAACAGTGTAAAACGACGGCCAGTGAGAT
BC07_2_b 
ATCTCACTGGCCGTCGTTTTACACTGTTCCACCAATTTGCATCGAGATGGTTACTCTG
CA
BC44_1_a
**G**AGTAAAAATCTCGATGCAAATTGGTGGAACAGTGTAAAACGACGGCCAGTGAGAT
BC44_1_b 
ATCTCACTGGCCGTCGTTTTACACTGTTCCACCAATTTGCATCGAGATTTTTACTCTG
CA
BC44_2_a
**GAG**TAAATATCTCGATGCAAATTGGTGGAACAGTGTAAAACGACGGCCAGTGAGAT
BC44_2_b 
ATCTCACTGGCCGTCGTTTTACACTGTTCCACCAATTTGCATCGAGATATTTACTCTG
CA
BC45_1_a
**GAG**TAAACATCGCGATGCAAATTGGTGGAACAGTGTAAAACGACGGCCAGTGAGAT
BC45_1_b 
ATCTCACTGGCCGTCGTTTTACACTGTTCCACCAATTTGCATCGCGATGTTTACTCTG
CA
BC45_2_a
**GAG**TAAACATCCCGATGCAAATTGGTGGAACAGTGTAAAACGACGGCCAGTGAGAT
BC45_2_b 
ATCTCACTGGCCGTCGTTTTACACTGTTCCACCAATTTGCATCGGGATGTTTACTCTG
CA
BC46_1_a
**GAG**TAAACATCTAGATGCAAATTGGTGGAACAGTGTAAAACGACGGCCAGTGAGAT
BC46_1_b 
ATCTCACTGGCCGTCGTTTTACACTGTTCCACCAATTTGCATCTAGATGTTTACTCTG
CA
BC46_2_a
**GAG**TAAACATCTGGATGCAAATTGGTGGAACAGTGTAAAACGACGGCCAGTGAGAT
BC46_2_b 
ATCTCACTGGCCGTCGTTTTACACTGTTCCACCAATTTGCATCCAGATGTTTACTCTG
CA
BC47_1_a
**GAG**TAAACATCTCGATGCAAATTGGTAGAACAGTGTAAAACGACGGCCAGTGAGAT
BC47_1_b

ATCTCACTGGCCGTCGTTTTACACTGTTCTACCAATTTGCATCGAGATGTTTACTCTG
CA

3. Method according to claim 1 **characterized by** the artificial polymorphic sites which are created as listed in Table 1.

4. Method according to claim 1 **characterized by** plasmid DNA obtained in the form of 10 plasmids:
1.pGAE-CAG-GFP-BC1 (abbrevation pBC1)
2. pGAE-CAG-GFP-BC2 (abbrevation pBC2)
3. pGAE-CAG-GFP-BC3 (abbrevation pBC3)
4. pGAE-CAG-GFP-BC4 (abbrevation pBC4)
5. pGAE-CAG-GFP-BC5 (abbrevation pBC5)
6. pGAE-CAG-GFP-BC6 (abbrevation pBC6)
7. pGAE-CAG-GFP-BC7 (abbrevation pBC7)
8. pGAE-CAG-GFP-BC8 (abbrevation pBC8)
9. pGAE-CAG-GFP-BC9 (abbrevation pBC9)
10. pGAE-CAG-GFP-BC10 (abbrevation pBC10)

5. The method according to claim 1 characterized that it is used for detecting variants of genetic barcodes introduced into cells / tissues or organisms.

6. The method according to claim 1 **characterized in that** it is used to track selected cell populations at the time and / or the progeny of a single cell.

7. The method according to claim 1 **characterized in that** it serves to study clonal evolution during the process of tumorgenesis and / or cells tracking during both in vitro and in vivo differentiation and organogenesis processes.

8. The usage of the method described in claim 1 for detecting variants of genetic barcodes introduced into cells / tissues or organisms.

9. The usage of the method depicted in claim 1 for tracing selected cell populations at time and / or progeny of a single cell.

10. The usage of the method described in claim 1 for studying clonal evolution during the process of tumorgenesis and / or cells tracking during both *in vitro* and *in vivo* differentiation and organogenesis processes.
